# EUROPÄISCHE PATENTANMELDUNG

(11) **EP 3 598 985 A1**
(43) Veröffentlichungstag der Anmeldung: **29.01.2020**
(21) Anmeldenummer: 18185022.3
(22) Anmeldetag: 23.07.2018
(51) Int. Cl.: A61L 2/18, A61L 2/20, A61C 19/06, A61C 17/02

(54) **DESINFEKTIONSMUNDSTÜCK**

(71) Anmelder: Herrmann Apparatebau GmbH, 63820 Elsenfeld (DE)
(72) Erfinder: HERRMANN, Wolfgang, 63820 Elsenfeld (DE)
(74) Vertreter: Götz, Georg Alois

(57) **Zusammenfassung**

Die Erfindung betrifft ein Mundstück (100) zur Desinfizierung der Mundhöhle mittels eines Fluids, insbesondere mittels eines Ozon-Sauerstoffgemischs, wobei das Mundstück (100) eine Fluidkammer (130) zur Aufnahme des Fluids umfasst und die Fluidkammer (130) mindestens einen Fluideinlass (121) zur Verbindung mit einer Fluidzuführleitung (210) und eine Vielzahl an Fluidauslässen (111) zur Beaufschlagung der Mundhöhle mit dem Fluid aufweist. Das Mundstück (100) ist zweiteilig ausgebildet, wobei ein erstes Mundstückteil (110), welches einer oberen oder einer unteren Zahnreihe der Mundhöhle zuordenbar ist und ein zweites Mundstückteil (120), welches der jeweils anderen, oberen oder unteren Zahnreihe der Mundhöhle zuordenbar ist, gemeinsam die Fluidkammer (130) begrenzen.

## Beschreibung

Die Erfindung betrifft ein Mundstück zur Desinfizierung der Mundhöhle mittels eines Fluids, insbesondere mittels eines Ozon-Sauerstoff-Gemischs, wobei das Mundstück eine Fluidkammer zur Aufnahme des Fluids umfasst und die Fluidkammer mindestens einen Fluideinlass zur Verbindung mit einer Fluidzuführleitung und eine Vielzahl an Fluidauslässen zur Beaufschlagung der Mundhöhle mit dem Fluid aufweist.

Die Erfindung betrifft ferner ein Desinfektionsset zur Desinfizierung der Mundhöhle mittels eines Fluids, insbesondere eines Ozon-Sauerstoff-Gemischs, mit einem eine Fluidkammer aufweisenden Mundstück, insbesondere einem erfindungsgemäßen Mundstück, wobei die Fluidkammer einen Fluideinlass sowie eine Vielzahl an Fluidauslässen zur Beaufschlagung der Mundhöhle mit dem Fluid aufweist, und einer Fluidzuführleitung, welche über ein erstes Leitungsende fluidleitend mit dem Fluideinlass verbunden ist und zur Zufuhr des Fluids in die Fluidkammer vorgesehen ist.

Im medizinischen und gerade im dentalmedizinischen Bereich wird neben konventionellen Behandlungsmethoden mehr und mehr auch auf nicht invasive Behandlungen von Mund-, Rachen- oder Zahn- bzw. Zahnfleischerkrankungen mittels desinfizierender Fluide, in Form von Gasgemischen oder Lösungen, zurückgegriffen. Als vielversprechend hat sich hierbei der Einsatz von Ozon erwiesen. Zweckmäßigerweise wird das zur Behandlung erforderliche Ozon mittels eines Ozongenerators direkt aus der Umgebungsluft gewonnen und als Ozon-Sauerstoffgemisch zur Desinfizierung der Mundhöhle zugeführt. Genutzt werden hierbei die desinfizierenden und keimabtötenden Eigenschaften des Ozons um unerwünschte Bakterien, Pilze und Viren abzutöten. In der Zahnmedizin lassen sich derart beispielsweise kariöse Erkrankungen ohne Bohren behandeln. Alternativ kann das Ozon-Sauerstoffgemisch ergänzend zur konventionellen Behandlung eingesetzt werden, beispielsweise zur Desinfizierung nach vorheriger Entfernung der kariösen Substanz mittels Bohrer, aber auch zur Desinfizierung oder Stabilisierung von Wurzelkanälen oder Zahnflächen. Für die Behandlung von Zahnfleischentzündungen und Parodontitis sowie zur antimikrobiellen Mundspülung lässt sich Ozon auch in flüssigem Aggregatszustand zuführen, z.B. in Form von mit Ozon angereichertem Wasser.

Neben der lokalen Behandlung von Karies- oder Entzündungsherden, bei welchen die Ozonanwendung auf eine kurzfristige Applikation an der betreffenden Stelle beschränkt ist, hat sich gerade zur präventiven Anwendung sowie zur Prophylaxe eine flächendeckende Beaufschlagung der Mundhöhle bzw. des Mundraums und der Zahnreihen eines Patienten als besonders wirkungsvoll herausgestellt.

Aus der DE 10 2010 011 080 A1 ist beispielsweise ein Mundstück bekannt, welches geeignet ist, den ganzen Mund in einem Arbeitsgang mit Ozon zu desinfizieren. Das Mundstück umfasst hierzu eine Maske eine vor den Lippen zu platzierende Maske um sowohl den Mund- als auch den Lippenbereich abzudecken. Zur Behandlung wird das Mundstück an ein Gerät angeschlossen, welches einen Ozongenerator sowie eine Absaugungspumpe aufweist. Das im Ozongenerator erzeugte Ozon wird über einen die Maske und den Maskenraum durchlaufenden Ozonzufuhrschlauch direkt der Mundhöhle zugeführt, wobei sich der Ozonzufuhrschlauch innerhalb der Mundhöhle T-artig gabelt. Die beiden Arme der T-artigen Gabelung sind jeweils zwischen den Zahnreihen und der Innenwange angeordnet, sodass der Ozonstrom im Backenzahnbereich in den Mundraum gelangt. Über einen Saugschlauch, welcher kurz hinter der Maske, innerhalb des Maskenraums und außerhalb des Mundraums, endet, wird verbleibendes Restozon abgesaugt. Nachteilig an der bekannten Vorrichtung ist, dass das Ozon ausschließlich an zwei Punkten, lokal im Bereich der Backenzähne appliziert wird, wodurch eine zuverlässige Beaufschlagung der übrigen Zähne, insbesondere der vollständigen Zahnreihen nicht sichergestellt werden kann. Aufgrund der vorgesehenen Absaugung wird außerdem ein Großteil des Ozons bereits aus dem Mundraum abgesaugt, bevor dieses in die Zahnzwischenräume eindringen und so die Zahnhälse oder darunterliegende Wurzeln erreichen kann. Bei der Verwendung von Umgebungsluft zur Erzeugung des erforderlichen Ozons können außerdem in der Umgebungsluft vorhandene, gegebenenfalls schädliche Bakterien oder Keime in die Mundhöhle des Patienten gelangen.

Ein ähnliches Mundstück, jedoch ohne Maske ist in der DE 10 2009 018 456 A1 offenbart. Um anstelle des gesamten Mundraums die Behandlung auf die beiden Zahnreihen und das jeweilige Zahnfleisch zu beschränken, weist das Mundstück zwei, hier als Kammern bezeichnete schalenartige Aufnahmen auf, welche durch eine Zwischenwand voneinander separiert sind und jeweils eine (obere oder untere) Zahnreihe umgeben bzw. einfassen. Über eine Leitung wird das Ozon in eine der Aufnahmeschalen geleitet und strömt links und rechts innerhalb der Aufnahmeschale von vorne nach hinten an den darin befindlichen Schneide- und Backenzähnen entlang. Über einen Durchbruch, welcher hinten, an den Enden der Zahnreihen angeordnet ist, strömt das Ozon in die zweite Aufnahmeschale, die wiederum mit der Absaugleitung verbunden ist. Auf diese Weise strömt das Ozon in der zweiten Aufnahmeschale von hinten nach vorne, sodass beide Zahnreihen gleichzeitig mit Ozon behandelt werden können. Auch bei dieser Vorrichtung ist der Ozonaustritt punktuell einer lokalen Stelle zugeordnet. Die übrigen Bereiche der Zahnreihen werden lediglich von dem daran vorbeiströmenden Ozon erfasst, sodass eine zuverlässige und vollständige Beaufschlagung beider Zahnreihen inklusive der Zahnzwischenräume sowie der darunterliegenden Zahnhälse und- wurzeln auch hier nicht möglich ist.

Auch aus der WO 2009/027845 A2 ist ein Mundstück zur dentalmedizinischen Behandlung mit einem Ozon-Sauerstoffgemisch bekannt. Das Mundstück ist aus einem einzelnen, flexiblen Silikonteil mit einer innenliegenden Kammer ausgebildet, welches zur Behandlung einer Zahnreihe mit einer Mikroperforierung versehen ist. Die Mikroperforierung bildet hierbei eine Vielzahl an Gasauslässen aus, sodass das zur Behandlung vorgesehene Ozon-Sauerstoffgemisch an den einzelnen Gasauslässen entlang der gesamten Zahnreihe austreten kann. Um das erforderliche Ozon-Sauerstoffgemisch zunächst in die Kammer einzubringen ist ein Gaseintritt mit einem angeschlossenen Schlauch vorgesehen. Zur Behandlung beider Zahnreihen gleichzeitig können wahlweise auch zwei Silikonteile mit jeweils einer innenliegenden Kammer verwendet werden. Nachteilig an der vorgeschlagenen einteiligen Ausführung, aus einem einzelnen, flexiblen Silikonteil ist, dass das Mundstück lediglich auf den Zahnoberflächen aufliegt, wodurch die Zahnreihen sowie das Zahnfleisch nicht vollständig von dem Mundstück umgeben bzw. eingefasst werden.

Angesichts des aufgezeigten Stands der Technik bietet sich im Hinblick auf Mundstücke sowie Desinfektionssets zur Desinfizierung der Mundhöhle eines Patienten noch Raum für Verbesserungen. Der Erfindung liegt daher die Aufgabe zugrunde, ein verbessertes Mundstück sowie ein verbessertes Desinfektionsset zur Desinfizierung der Mundhöhle eines Patienten bereitzustellen. Insbesondere soll die Verteilung des Fluids innerhalb der Zahnzwischenräume sowie zu den Zahnhälsen verbessert und die Einbringung zusätzlicher Keime oder Bakterien durch das zur Behandlung zugeführte Fluid vermieden werden.

Erfindungsgemäß wird die Aufgabe durch ein Mundstück mit den Merkmalen des Anspruchs 1 sowie ein Desinfektionsset mit den Merkmalen des Anspruchs 8, welches insbesondere ein erfindungsgemäßes Mundstück aufweist, gelöst. Weitere vorteilhafte Ausgestaltungen der Erfindung sind in den jeweiligen Unteransprüchen offenbart.

Es ist darauf hinzuweisen, dass die in der nachfolgenden Beschreibung einzeln aufgeführten Merkmale sowie Maßnahmen in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Ein erfindungsgemäßes Mundstück der eingangs näher bezeichneten Art kennzeichnet sich dadurch, dass das Mundstück zweiteilig ausgebildet ist, wobei ein erstes Mundstückteil, welches einer oberen oder einer unteren Zahnreihe der Mundhöhle zuordenbar ist und ein zweites Mundstückteil, welches der jeweils anderen, oberen oder unteren Zahnreihe der Mundhöhle zuordenbar ist, gemeinsam die Fluidkammer begrenzen.

Erfindungsgemäß ist das Mundstück somit zweiteilig bzw. zweistückig ausgebildet und weist ein erstes Mundstückteil sowie ein zweites Mundstückteil auf, welche gemeinsam die innenliegende, insbesondere einzige Fluidkammer ausbilden. Die Fluidkammer ist also innerhalb der bzw. zwischen den beiden Mundstückteilen angeordnet und wird von diesen begrenzt, wobei die beiden Mundstückteile vorzugsweise fluiddicht und insbesondere nicht lösbar, miteinander verbunden sind. Zur Behandlung, d.h. zur Desinfizierung des Mundraums und der Zahnreihen mittels des Fluids, insbesondere mittels des Ozon-Sauerstoffgemischs bzw. zur Beaufschlagung des Mundraums und der Zahnreihen mit dem Fluid, insbesondere mit dem Ozon-Sauerstoffgemisch wird das Mundstück innerhalb des Mundraums bzw. der Mundhöhle eines Patienten platziert und liegt auf den Zahnreihen auf. Zweckmäßigerweise sind das gesamte Mundstück sowie die beiden Mundstückteile hierzu hufeisenartig geformt, um dem natürlichen Verlauf des Zahnbogens zu folgen. Wahlweise wird eines der beiden Mundstückteile der oberen und das entsprechend andere Mundstückteil der unteren Zahnreihe zugeordnet. Das zur Desinfizierung erforderliche Fluid, bspw. ein Ozon-Sauerstoffgemisch kann durch einen aus dem Stand der Technik bekannten Ozongenerator aus der Umgebungsluft gewonnen und bereit gestellt werden. Zweckmäßiger ist es jedoch, wenn das Ozon-Sauerstoffgemisch aus medizinischem Sauerstoff gewonnen wird, welcher in Druckflaschen käuflich erhältlich ist. Der medizinische Sauerstoff wird dem OzonGenerator zugeführt und das erzeugte Ozon-Sauerstoffgemisch bspw. in Einmalspritzen abgefüllt. Das in der Einmalspritze enthaltene Gemisch kann dann unmittelbar oder mittelbar über eine Fluidzuführleitung, insbesondere einen Schlauch, dem Fluideinlass des Mundstücks zugeführt werden. Über den Fluideinlass tritt das Fluid, insbesondere das Ozon-Sauerstoffgemisch in die Fluidkammer ein und kann diese aufgrund der vorzugsweise fluiddichten Verbindung zwischen dem ersten Mundstückteil und dem zweiten Mundstückteil ausschließlich über die Vielzahl an Fluidauslässen verlassen. Zweckmäßigerweise sind die Fluidauslässe der oder den zu behandelnden Zahnreihen zugeordnet und entlang dieser verteilt.

Aufgrund der zweiteiligen bzw. zweistückigen Ausführung des Mundstücks ist es möglich, das erste Mundstückteil und das zweite Mundstückteil mit voneinander abweichenden Materialeigenschaften, beispielsweise mit voneinander abweichender Flexibilität, d.h. Steifigkeit und/oder Starrheit, oder sogar aus unterschiedlichen Materialien herzustellen. Vorzugsweise sind beide Mundstückteile des Mundstücks aus einem spritzgussfähigen Material, insbesondere aus einem thermoplastischen Elastomer hergestellt und weisen eine ähnliche Shore-Härte, in einem Bereich zwischen 60-80 Shore, besonders bevorzugt in etwa 70 Shore auf. Um eine unterschiedliche Flexibilität oder unterschiedliche Verformungseigenschaften zu erhalten, können die Mundstückteile mit unterschiedlicher Materialstärke ausgeführt werden. Die Gesamtgröße des Mundstücks kann an den zu behandelnden Patienten angepasst werden und beispielsweise in drei Ausführungen, groß, mittel und klein bereit gestellt werden.

Die zweiteilige Ausführung weist außerdem Vorteile im Herstellungsverfahren auf, da beide Mundstückteile separat im Spritzgussverfahren hergestellt werden können und anschließend zur Ausbildung der innenliegenden Kammer zusammengesetzt werden. Dies ermöglicht eine einfache und kostengünstige Herstellung.

In einer vorteilhaften Ausgestaltung des erfindungsgemäßen Mundstücks ist das erste Mundstückteil zumindest teilweise innerhalb des zweiten Mundstückteils angeordnet, wobei das erste Mundstückteil einen Aufnahmebereich zur Aufnahme der zugeordneten, oberen oder unteren, Zahnreihe begrenzt.

Insbesondere weisen in dieser Ausgestaltung sowohl das erste, innenliegende als auch das zweite, außenliegende Mundstückteil eine hufeisenartige Grundform mit einem U-förmigen und/oder V-förmigen Querschnitt bzw. Profil auf. Auf diese Weise kann die dem ersten, innenliegenden Mundstückteil zugeordnete Zahnreihe innerhalb des Aufnahmebereichs angeordnet werden, sodass die Zahnreihe zur Beaufschlagung mit dem Ozon-Sauerstoffgemisch von dem ersten, innenliegenden Mundstückteil umgeben bzw. eingefasst wird. Zur Behandlung kann also entweder die obere Zahnreihe oder die unteren Zahnreihe des Patienten, je nachdem, welche der beiden Zahnreihen unmittelbar mit dem Ozon-Sauerstoffgemisch beaufschlagt werden soll, innerhalb des Aufnahmebereichs des Mundstücks bzw. innerhalb des durch das innenliegende, erste Mundstückteil ausgebildeten Aufnahmebereichs angeordnet werden.

Eine ebenso vorteilhafte Ausgestaltung der Erfindung wird erzielt, indem das zweite Mundstückteil mit einer gegenüber dem ersten Mundstückteil geringeren Flexibilität, als, insbesondere außenliegende, Verschalung ausgebildet ist und das erste Mundstückteil zumindest teilweise mit einer gegenüber dem zweiten Mundstückteil höheren Flexibilität als, insbesondere innenliegende, Membran ausgebildet ist.

Durch die Ausbildung des zweiten Mundstückteils als Verschalung mit einer gegenüber dem ersten Mundstückteil geringeren Flexibilität, d.h. einer höheren Steifigkeit und/oder einer höheren Starrheit, kann dieses maßgeblich zur Formgebung und Stabilisierung des Mundstücks genutzt werden. Gleichzeitig ist vorgesehen, dass das zweite Mundstückteil weiterhin zumindest geringe Verformungsmöglichkeiten und/oder eine geringe Elastizität aufweist, um eine Anpassung an die anatomischen Vorgaben der Mundhöhle bzw. des Mundraums des Patienten zu ermöglichen, was außerdem zu einem komfortableren und schmerzfreien Sitz des Mundstücks innerhalb der Mundhöhle beiträgt. Zur Beaufschlagung der Mundhöhle und der Zahnreihen mit dem Fluid, insbesondere dem Ozon-Sauerstoffgemisch, liegt das zweite Mundstückteil bspw. auf der unteren Zahnreihe des Patienten auf und ist vorteilhafter Weise in dessen Formgebung an die entsprechenden Zahnoberfläche der Zahnreihe angepasst. Aufgrund des vorzugsweisen V-förmigen und/oder U-förmigen Profils wird die, in diesem Fall obere Zahnreihe von dem Mundstück umgeben bzw. eingefasst.

Das zumindest teilweise als Membran, mit einer gegenüber dem zweiten Mundstückteil höheren Flexibilität, d. h. geringeren Steifigkeit und/oder geringeren Starrheit, ausgebildete erste Mundstückteil ist vorzugsweise innerhalb des zweiten Mundstückteils angeordnet und begrenzt derart einen Aufnahmebereich zur Aufnahme der oberen Zahnreihe. Durch die Ausbildung des ersten Mundstückteils als Membran, passt sich dieses flexibel der Form der oberen Zahnreihe an, sodass das erste Mundstückteil die obere Zahnreihe umschließt oder sogar zumindest abschnittsweise an den Kauflächen und/oder den seitlichen Zahnoberflächen anliegt.

Zur Ausbildung der unterschiedlichen Flexibilität, d.h. der unterschiedlichen Steifigkeit und/oder Starrheit können das erste Mundstückteil und das zweite Mundstückteil beispielsweise aus demselben Material, mit einer identischen Härte, insbesondere Shore-Härte, jedoch mit unterschiedlicher Materialstärke hergestellt sein. Insbesondere weist das erste, als Membran ausgebildete Mundstückteil eine wesentlich geringere Dicke bzw. Wandstärke oder Materialstärke als das zweite, als Verschalung ausgebildete Mundstückteil auf. Eine zumindest geringfügige Verformbarkeit auch des zweiten, als Verschalung ausgebildeten Mundstückteils kann durch Festlegung einer entsprechenden Härte erlangt werden. Für ein thermoplastisches Elastomer hat sich hierzu eine Shore-Härte in einem Bereich zwischen 60-80-Shore, besonders bevorzugt eine Härte von etwa 70 Shore herausgestellt.

Darüber hinaus hat sich eine Ausführungsform des erfindungsgemäßen Mundstücks als vorteilhaft erwiesen, bei welcher das erste Mundstückteil mit einer Perforierung versehen ist, wobei die Perforierung die Vielzahl an Fluidauslässen ausbildet.

Insbesondere, wenn das erste Mundstückteil zumindest teilweise als Membran ausgeführt ist, können die Fluidauslässe in einfacher Weise durch eine Perforierung der Membran ausgeführt sein. Vorzugsweise verlaufen die Fluidauslässe bzw. verläuft die Perforierung entlang des Aufnahmebereichs des ersten Mundstückteils, sodass die zugeordnete Zahnreihe in direktem Kontakt mit der perforierten Oberfläche und derart mit den Fluidauslässen des ersten Mundstückteils steht. Auf diese Weise kann das Fluid, insbesondere das Ozon-Sauerstoffgemisch gezielt auch an schwer zugänglichen Stellen der Zahnreihe wie beispielsweise Zahnzwischenräumen oder Zahnhälsen appliziert werden. Die Strömungsrichtung des Fluids ist beim Verlassen der Fluidauslässe in Richtung der Zahnreihe sowie in Richtung der Kaufflächen bzw. der Zahnhälse gerichtet. Indem das Fluid nicht lediglich an der Zahnreihe vorbeiströmt, sondern dessen Strömung direkt auf die Zahnreihe gerichtet ist, kann einerseits eine vollständige Beaufschlagung des Mundraums und andererseits auch eine zuverlässige Desinfizierung der Zahnzwischenräume sowie der Zahnhälse sichergestellt werden.

Um eine gleichmäßige Beaufschlagung der vorderen, der Schneide- und Eckzähne sowie der hinteren, der Backenzähne mit dem Fluid zu gewährleisten hat es sich in Weiterbildung als zweckmäßig erwiesen, die Perforierung mit einer in Richtung der Fluidströmung zunehmenden Lochgröße auszubilden. Insbesondere weisen die dem Fluideinlass benachbarten und somit den vorderen Zähnen zugeordneten Fluidauslässe eine kleinere Querschnittsfläche auf, als die von dem Fluideinlass weiter beabstandeten und somit den hinteren Zähnen zugeordneten Fluidauslässe. Durch einen derartigen Lochgrößenverlauf kann der innerhalb der Fluidkammer auftretende Strömungsverlust ausgeglichen werden.

Bei einer vorteilhaften Ausführungsvariante des erfindungsgemäßen Mundstücks sind das erste Mundstückteil und das zweite Mundstückteil mittels einer Feder-Nut-Verbindung fluiddicht miteinander verbunden oder verbindbar.

Vorzugsweise weist das als Verschalung ausgebildete zweite Mundstückteil eine Kante oder Feder, insbesondere eine umlaufende Kante oder Feder auf, in welche das als Membran ausgebildete erste Mundstückteil eingesteckt oder einsteckbar bzw. eingeschnappt oder einschnappbar ist. Das erste Mundstückteil kann hierzu einen außenliegenden Verbindungsbereich, welcher mit einer höheren Materialstärke als der innenliegende Membranbereich ausgeführt ist aufweisen. Der Verbindungsbereich ist dann weniger flexibel, d. h. steifer und/oder starrer und mit einer höheren Stabilität ausgeführt, sodass sich dieser besonders zur Ausbildung einer Vertiefung oder Nut, in welche die Kante oder Feder des zweiten Mundstückteil einsteckbar bzw. einschnappbar ist, eignet. Die Verbindung kann lösbar ausgeführt sein und ermöglicht eine ausreichende Fluiddichtigkeit, damit das Fluid, insbesondere das Ozon-Sauerstoffgemisch die Fluidkammer ausschließlich durch die hierzu vorgesehenen Fluidauslässe verlässt. Vorzugsweise sind das erste Mundstückteil und das zweite Mundstückteil nicht lösbar miteinander verbunden und beispielsweise alternativ oder zusätzlich miteinander verklebt.

Es ist außerdem vorteilhaft, wenn das erste Mundstückteil und das zweite Mundstückteil mittels die Fluidkammer durchsetzender Trennstege voneinander beabstandet angeordnet sind.

Gerade während der Verwendung, wenn sich das Mundstück zur Behandlung innerhalb der Mundhöhle eines Patienten befindet, könnte es bspw. durch Zubeißen zu unerwünschten Verformungen des ersten und/oder des zweiten Mundstückteils kommen, wodurch eine Verschluss der Fluidkammer oder eine Abtrennung eines oder mehrerer Bereiche der Fluidkammer auftreten kann. Um derartige unerwünschte Verformungen zu vermeiden können zwischen dem ersten Mundstückteil und dem zweiten Mundstückteil Trennstege angeordnet, welche bspw. entweder mit dem ersten oder dem zweiten Mundstückteil verbunden sind und mit dem jeweils anderen Mundstückteil einen Anschlag bilden. Auf diese Weise wird verhindert, dass die beiden Mundstückteile durch Krafteinwirkung, bspw. durch Zubeißen des Patienten einander berühren und so zu einem Verschluss der Fluidkammer oder einer Abtrennung eines Bereichs der Fluidkammer führen.

Schließlich weist gemäß einer bevorzugten Ausführungsvariante der Erfindung das zweite Mundstückteil den Fluideinlass auf, wobei der Fluideinlass zum Anschluss einer Fluidzuführleitung oder Fluidzufuhr ausgebildet ist.

Gerade, wenn das zweite Mundstückteil als Verschalung, mit einer geringeren Flexibilität gegenüber dem ersten Mundstückteil ausgebildet ist, ist es zweckmäßig den Fluideinlass durch das zweite Mundstückteil auszubilden. Auf diese Weise weist der vorzugsweise einstückig mit dem zweiten Mundstückteil ausgeführte Fluideinlass eine ausreichende Steifigkeit und/oder Starrheit auf, um als Anschluss für eine Fluidzuführleitung oder Fluidzufuhr, insbesondere einen Schlauch zu fungieren. Alternativ ist es auch denkbar das zuzuführenden Fluid mittels einer Spritze direkt in den Fluideinlass zu applizieren.

Ein erfindungsgemäßes Desinfektionsset zur Desinfizierung der Mundhöhle mittels eines Fluids, insbesondere eines Ozon-Sauerstoffgemischs, der eingangs näher bezeichneten Art welches ein Mundstück, insbesondere gemäß einer oder mehrerer der zuvor beschriebenen Ausführungsformen und eine Fluidzuführleitung aufweist, kennzeichnet sich durch ein Filterelement, welches zur Filterung des Fluids, insbesondere des Ozon-Sauerstoff-Gemischs, mit einem zweiten Leitungsende der Fluidzuführleitung verbunden ist.

Erfindungsgemäß ist also vorgesehen, dass die Fluidzuführleitung an ihrem ersten Leitungsende das Mundstück aufweist und an ihrem zweiten Leitungsende mit einem Filterelement verbunden ist. Durch Verwendung eines Filterelements, insbesondere eines Einmal-Bakterienfilters kann unter anderem vermieden werden, dass zusätzliche Keime oder Bakterien bei der Einbringung des Fluids, insbesondere des Ozon-Sauerstoffgemischs in die Mundhöhle bzw. den Mundraum eines Patienten eingebracht werden. Das Filterelement ist hierzu in der Fluidzuführleitung angeordnet, wobei das Fluid in Strömungsrichtung vor dem Filter entweder zunächst der Fluidzuführleitung oder direkt dem Filter zugeführt wird, diesen durchströmt und hierbei von Bakterien und/oder Keimen gefiltert wird, um anschließend am ersten Leitungsende der Fluidzuführleitung durch den Fluideinlass in die Fluidkammer des Mundstücks einzutreten.

Bei einer bevorzugten Ausführungsform des erfindungsgemäßen Desinfektionssets weist die Fluidzuführleitung oder das Filterelement hierzu einen Anschluss, insbesondere zum Anschließen einer Spritze auf, wobei das Fluid über den Anschluss in die Zuführleitung des Desinfektionssets einleitbar und das Filterelement durchströmend der Fluidkammer des Mundstücks zuführbar ist.

Durch die Abgabe der erforderlichen Fluidmenge mittels einer Spritze kann beispielsweise auch ein Überschreiten der zulässigen Ozonmenge vermieden werden.

Nach einer bevorzugten Ausführung ist das Mundstück zweiteilig ausgebildet, wobei ein erstes Mundstückteil, welches einer oberen oder einer unteren Zahnreihe der Mundhöhle zuordenbar ist und ein zweites Mundstückteil, welches der jeweils anderen, oberen oder unteren Zahnreihe der Mundhöhle zuordenbar ist, die Fluidkammer begrenzen.

In Weiterbildung ist es vorteilhaft, wenn das erste Mundstückteil zumindest teilweise als Membran ausgebildet ist und die Vielzahl an Fluidauslässen aufweist, und das zweite Mundstückteil als Verschalung ausgebildet ist und den Fluideinlass aufweist, wobei das erste Leitungsende der Fluidzuführleitung in die Fluidkammer mündend mit dem Fluideinlass verbunden ist.

Vorzugsweise sind also das zweite Mundstückteil, die Fluidzuführleitung, insbesondere der Schlauch und das Filterelement, insbesondere der Einmalbakterienfilter fest miteinander verbunden und/oder verklebt. Das erste Mundstückteil lässt sich mittels einer Nut-Feder-Verbindung mit dem zweiten Mundstückteil fluiddicht verbinden und/oder verkleben, um die Fluidkammer auszubilden.

Weitere vorteilhafte Ausgestaltungen der Erfindung sind in der folgenden Figurenbeschreibung offenbart. Es zeigen
- Fig. 1: eine perspektivische Darstellung einer beispielhaften Ausführungsform eines erfindungsgemäßen Mundstücks,
- Fig. 2: eine perspektivische Schnittansicht der beispielhaften Ausführungsform des erfindungsgemäßen Mundstücks aus Figur 1,
- Fig. 3: eine perspektivische Darstellung eines ersten Mundstückteils der beispielhaften Ausführungsform des erfindungsgemäßen Mundstücks aus Figur 1,
- Fig. 4: eine perspektivische Darstellung eins zweiten Mundstückteils der beispielhaften Ausführungsform des erfindungsgemäßen Mundstücks aus Figur 1,
- Fig. 5: eine perspektivische Darstellung einer beispielhaften Ausführungsform eines erfindungsgemäßen Desinfektionssets mit einem Mundstück, einer Fluidzuführleitung und einem Filterelement.

In den unterschiedlichen Figuren sind gleiche Teile stets mit denselben Bezugszeichen versehen, weswegen diese in der Regel auch nur einmal beschrieben werden.

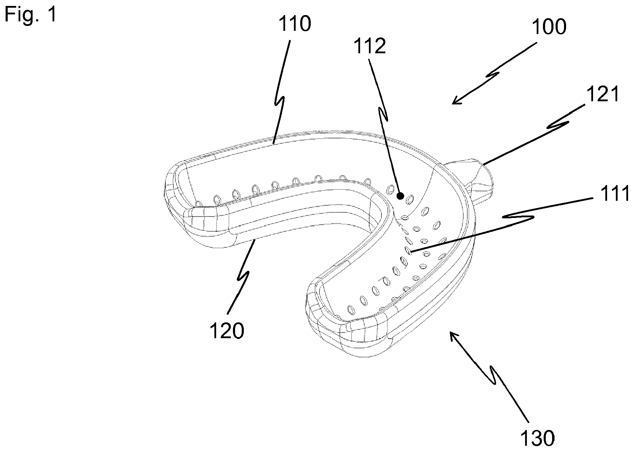

Der Figur 1 kann eine perspektivische Darstellung einer beispielhaften Ausführungsform eines erfindungsgemäßen Mundstücks 100 entnommen werden. Das Mundstück 100 weist eine im Wesentlichen hufeisenartige Form auf und besteht aus einem ersten Mundstückteil 110, auch als Membranteil bezeichnet, welches innerhalb eines zweiten Mundstückteils 120, auch als Unterschale bezeichnet, angeordnet ist. Das erste Mundstückteil 110 ist zumindest teilweise als Membran ausgebildet und umfasst eine Perforierung, welche eine Vielzahl an Fluidauslässen 111 ausbildet. Die Fluidauslässe 111 stehen mit einer innerhalb des Mundstücks 100 liegenden, hier nicht sichtbaren, Fluidkammer 130 in fluidleitender Verbindung. Das zweite Mundstückteil 120 weist einen Fluideinlass 121 auf, über welchen der Fluidkammer 130 ein Fluid, insbesondere ein zur Desinfizierung der Mundhöhle und der Zahnreihen eines Patienten geeignetes Ozon-Sauerstoffgemisch zugeführt werden kann, Anschließend kann das Fluid die Fluidkammer 130 über die Fluidauslässe 111 verlassen. Zur Beaufschlagung der Mundhöhle sowie einer Zahnreihe mit dem Fluid, wird das Mundstück 100 in der Mundhöhle des Patienten platziert, wobei die zu behandelnde Zahnreihe, bspw. die obere Zahnreihe, innerhalb eines Aufnahmebereichs 112, welcher durch das erste Mundstückteil 110 begrenzt ist, angeordnet wird. Das zweite Mundstückteil 120 liegt dann auf der unteren Zahnreihe des Patienten auf. Die Perforierung des als Membran ausgebildeten ersten Mundstückteils 110 ist derart realisiert, dass die Fluidauslässe 111 zur Beaufschlagung der seitlichen Zahnoberflächen sowohl seitlich, als auch zur Beaufschlagung der Kauflächen, oberhalb der Zahnreihe angeordnet sind. Auf diese Weise kann sichergestellt werden, dass das Fluid, insbesondere das Ozon-Sauerstoffgemisch auch die Zahnzwischenräume sowie die Zahnhälse erreicht und diese vollständig desinfiziert werden.

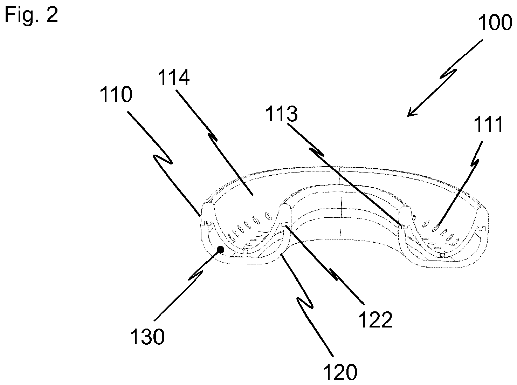

Zur Darstellung der zwischen dem ersten Mundstückteil 110 und dem zweiten Mundstückteil 120 ausgebildeten Fluidkammer 130 ist die beispielhafte Ausführungsform des erfindungsgemäßen Mundstücks 100 aus Figur 1 in der Figur 2 als Schnittdarstellung gezeigt. Zu erkennen ist, dass das erste Mundstückteil 110 und das zweite Mundstückteil 120 jeweils mit einem V-förmigen und/oder U-förmigen Profil ausgebildet sind, wobei das erste Mundstückteil 110 innerhalb des zweiten Mundstückteil ist 120 angeordnet ist und teilweise von diesem umgeben ist. Das außenliegende, zweite Mundstückteil 120 weist zur Verbindung mit dem ersten Mundstückteil 110 eine umlaufende Kante bzw. Feder 122, in welche eine komplementär ausgebildete, das erste Mundstückteil 110 umlaufende Ausnehmung oder Vertiefung bzw. Nut 113 aufgesteckt und/oder eingeschnappt ist. Um eine ausreichende Fluiddichtigkeit der Verbindung zwischen dem ersten Mundstückteil 110 und dem zweiten Mundstückteil 120 zu gewährleisten, kann die Nut-Feder-Verbindung zusätzlich verklebt sein. Zwischen den beiden Mundstückteilen 110, 120 ist die im Querschnitt ebenfalls V-förmig und/oder U-förmig ausgebildete Fluidkammer 130 angeordnet und wird von diesen begrenzt. Die Fluidkammer 130 wird somit sowohl durch das erste Mundstückteil 110, wie auch durch das zweite Mundstückteil 120 begrenzt.

Der Schnittdarstellung gemäß Figur 2 kann außerdem entnommen werden, dass das erste Mundstückteil 110 einen innenliegenden bzw. mittleren membranartigen Membranbereich 114 umfasst, welcher die Perforierung aufweist und dessen Materialstärke geringer ist, als die Materialstärke des als Verschalung ausgebildeten zweiten Mundstückteils 120. Durch die Verwendung unterschiedlicher Materialstärken kann eine unterschiedliche Flexibilität, d.h. Steifigkeit und/oder Starrheit der beiden Mundstückteile 110 und 120 - auch bei Verwendung eines identischen Materials, insbesondere thermoplastischen Elastomers - erlangt werden. Als geeignetes Ausgangsmaterial hat sich bspw. ein thermoplastisches Elastomer mit der Bezeichnung TPE MEDIPRENE® 880203 70 ShA mit einer Shore-Härte von 70 Shore herausgestellt.

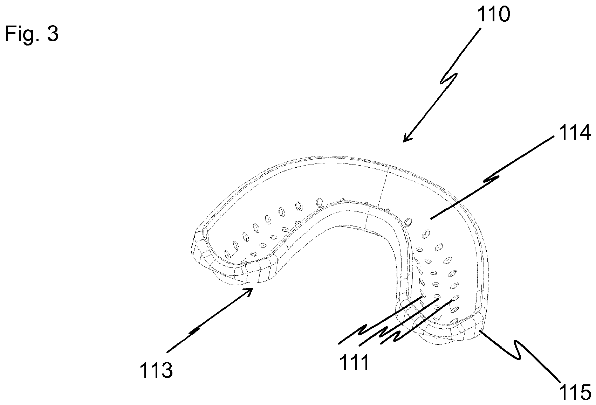

In der Figur 3 ist eine perspektivische Darstellung nur des ersten Mundstückteils 110 einzeln gezeigt. Die Fluidauslässe 111 sind hier beispielhaft in drei Reihen verlaufend entlang der hufeisenförmigen Erstreckung des ersten Mundstückteils 110 angeordnet, wobei eine mittlere Reihe den Kauflächen und jeweils eine rechts bzw. links zu dieser angeordnete, seitliche Reihe den entsprechenden seitlichen Zahnoberflächen der Zahnreihe eines Patienten zuordenbar ist. Das Mundstück 100 kann mit weiteren oder auch weniger Fluidauslässen 111 oder Reihen an Fluidauslässen 111 in beliebiger, für die vorgesehene Behandlung sinnvoller Weise ausgestaltet sein. Wie auch in der Schnittdarstellung gemäß Figur 2 zu erkennen, wird ein innenliegender, mit der Perforierung und den Fluidauslässen 111 versehener Membranbereich 114 des ersten Mundstückteils 110 durch einen außenliegenden Verbindungsbereich 115 eingefasst bzw. umschlossen. Der Verbindungsbereich 115 ist mit einer höheren Materialstärke als der Membranbereich 114 ausgeführt, weshalb dieser eine höhere Stabilität zur Aufnahme der umlaufenden Ausnehmung bzw. der Nut 113 aufweist.

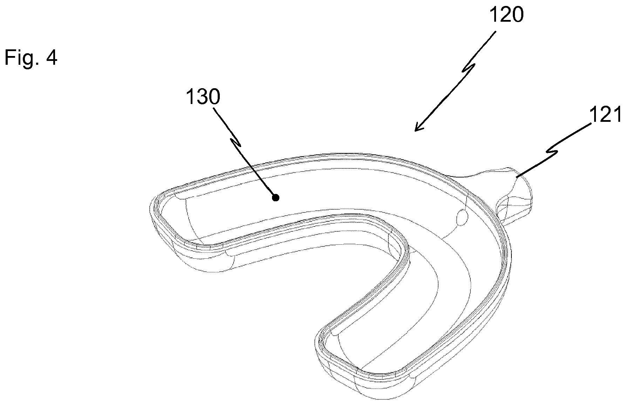

Das zweite Mundstückteil 120 der beispielhaften Ausführungsform des erfindungsgemäßen Mundstücks 100 aus Figur 1 ist in der Figur 4 separat dargestellt. Dieses ist im Wesentlichen hufeisenförmig und in der Art einer Verschalung mit einer gegenüber dem ersten Mundstückteil 110 geringeren Flexibilität, d.h. höheren Steifigkeit und/oder Starrheit ausgebildet. Das zweite Mundstückteil 120 weist einen einzelnen Fluideinlass 121 auf, welcher zur Verbindung mit bzw. zur Aufnahme eines Schlauchendes oder einer ähnlichen Anschlussleitung zur Zufuhr des Fluids vorgesehen ist der Fluideinlass 121 mündet in das als Verschalung ausgebildete zweite Mundstückteil 120, sodass das über diesen zuströmende Fluid in die Fluidkammer 130, welche durch Verbindung des ersten Mundstückteils 110 mit dem zweiten Mundstückteil 120 ausgebildet wird, einströmt. Das zweite Mundstückteil 120 weist keine weiteren Öffnungen auf, womit ein Austritt des Fluids ausschließlich durch die am ersten Mundstückteil 110 ausgebildeten Fluidauslässe 111 erfolgt.

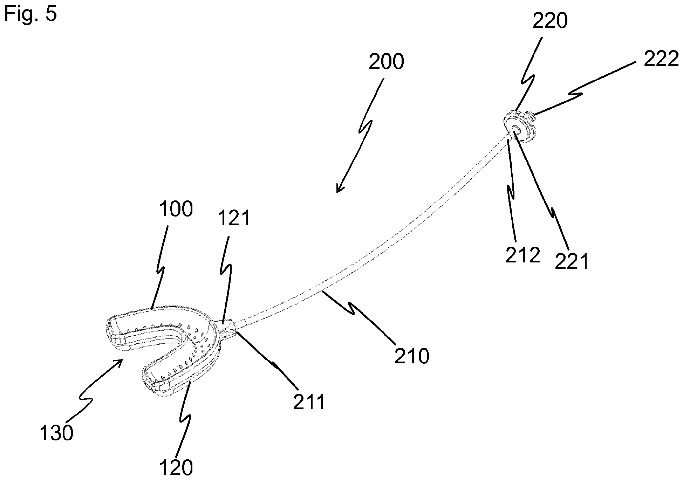

Schließlich kann der Figur 5 eine perspektivische Darstellung einer beispielhaften Ausführungsform eines erfindungsgemäßen Desinfektionssets 200 mit einem Mundstück 100, einer Fluidzuführleitung 210 und einem Filterelement 220. Das hier beispielhaft verwendete Mundstück 100 entspricht der in den vorangegangenen Absätzen beschriebenen Ausführungsform gemäß der Figuren 1-4 und wird daher nachfolgend nicht weiter erläutert. Ein erstes Leitungsende 211 der Fluidzuführleitung 210 ist mit dem Fluideinlass 121 des zweiten Mundstückteils 120 des Mundstücks 100 verbunden. Vorzugsweise ist die Fluidzuführleitung 210 als Schlauch ausgeführt und dessen erstes Leitungsende 211 in den Fluideinlass 121 eingesteckt. Zur Verbesserung der Fluiddichtigkeit zwischen der Fluidzuführleitung 210 und dem Mundstück 100 kann deren erstes Leitungsende 211 mit dem in die Fluidkammer 130 führenden Fluideinlass 121 verklebt sein. An einem zweiten Leitungsende 212 der Fluidzuführleitung 210 ist das Filterelement 220 angeordnet. Bei dem Filterelement 220 handelt es sich vorzugsweise um einen Einmalbakterienfilter, welcher zur Abfilterung von Bakterien und Keimen aus dem zugeführten Fluid geeignet ist. Zweckmäßigerweise ist das Filterelement 220 in das zweite Leitungsende 212 der Fluidzuführleitung 210 eingesteckt bzw. ist das zweite Leitungsende 212 der Fluidzuführleitung 210 auf einen, bspw. als Luer-Verbindung ausgeführten Verbindungsfortsatz 221 aufgeschoben und optional, zur Verbesserung der Fluiddichtigkeit, mit diesem verklebt. Dem Verbindungsfortsatz 221 gegenüberliegend auf der anderen Seite des Filterelements 220 angeordnet ist ein, insbesondere mit einem Außengewinde versehener Anschluss 222 vorgesehen, welcher beispielsweise zur Verbindung, insbesondere Verschraubung mit einer Einmalspritze oder einer sonstigen das Fluid, insbesondere das Ozon-Sauerstoffgemisch bereitstellenden Einrichtung vorgesehen ist.

Alle für das Desinfektionsset 200 verwendeten Materialien, einschließlich der beiden Mundstückteile 110, 120, der Fluidzuführleitung 210 sowie des Filterelements 220 sind ozonbeständig und für medizinische Anwendungszwecke geeignet.

### Bezugszeichenliste:

- 100: Mundstück
- 110: erstes Mundstückteil
- 111: Fluidauslass
- 112: Aufnahmebereich
- 113: Nut
- 114: Membranbereich
- 115: Verbindungsbereich
- 120: zweites Mundstückteil
- 121: Fluideinlass
- 122: Feder
- 130: Fluidkammer
- 200: Desinfektionsset
- 210: Fluidzuführleitung
- 211: erstes Leitungsende
- 212: zweites Leitungsende
- 220: Filterelement
- 221: Verbindungsfortsatz
- 222: Anschluss

## Patentansprüche

1. Mundstück (100) zur Desinfizierung der Mundhöhle mittels eines Fluids, insbesondere mittels eines Ozon-Sauerstoffgemischs, wobei das Mundstück (100) eine Fluidkammer (130) zur Aufnahme des Fluids umfasst und die Fluidkammer (130) mindestens einen Fluideinlass (121) zur Verbindung mit einer Fluidzuführleitung (210) und eine Vielzahl an Fluidauslässen (111) zur Beaufschlagung der Mundhöhle mit dem Fluid aufweist,
**dadurch gekennzeichnet, dass**
das Mundstück (100) zweiteilig ausgebildet ist, wobei ein erstes Mundstückteil (110), welches einer oberen oder einer unteren Zahnreihe der Mundhöhle zuordenbar ist und ein zweites Mundstückteil (120), welches der jeweils anderen, oberen oder unteren Zahnreihe der Mundhöhle zuordenbar ist, gemeinsam die Fluidkammer (130) begrenzen.

2. Mundstück (100) nach Anspruch 1,
**dadurch gekennzeichnet, dass**
das erste Mundstückteil (110) zumindest teilweise innerhalb des zweiten Mundstückteils (120) angeordnet ist, wobei das erste Mundstückteil (110) einen Aufnahmebereich (112) zur Aufnahme der zugeordneten, oberen oder unteren, Zahnreihe begrenzt.

3. Mundstück (100) nach Anspruch 1 oder 2,
**dadurch gekennzeichnet, dass**
das zweite Mundstückteil (120) mit einer gegenüber dem ersten Mundstückteil (110) geringeren Flexibilität, als Verschalung ausgebildet ist und das erste Mundstückteil (110) zumindest teilweise mit einer gegenüber dem zweiten Mundstückteil (120) höheren Flexibilität als Membran ausgebildet ist.

4. Mundstück (100) nach einem der vorhergehenden Ansprüche,
**dadurch gekennzeichnet, dass**
das erste Mundstückteil (120) mit einer Perforierung versehen ist, wobei die Perforierung die Vielzahl an Fluidauslässen (111) ausbildet.

5. Mundstück (100) nach einem der vorhergehenden Ansprüche,
**dadurch gekennzeichnet, dass**
das erste Mundstückteil (110) und das zweite Mundstückteil (120) mittels einer Feder-Nut-Verbindung fluiddicht miteinander verbunden oder verbindbar sind.

6. Mundstück (100) nach einem der vorhergehenden Ansprüche,
**dadurch gekennzeichnet, dass**
das erste Mundstückteil (110) und das zweite Mundstückteil (120) mittels die Fluidkammer (130) durchsetzender Trennstege voneinander beabstandet angeordnet sind.

7. Mundstück (100) nach einem der vorhergehenden Ansprüche,
**dadurch gekennzeichnet, dass**
das zweite Mundstückteil (120) den Fluideinlass (121) aufweist, wobei der Fluideinlass (121) zum Anschluss einer Fluidzuführleitung (210) ausgebildet ist.

8. Desinfektionsset (200) zur Desinfizierung der Mundhöhle mittels eines Fluids, insbesondere eines Ozon-Sauerstoff-Gemischs, mit
- einem eine Fluidkammer (130) aufweisenden Mundstück (100), insbesondere nach einem der Ansprüche 1-6, wobei die Fluidkammer (130) einen Fluideinlass (121) sowie eine Vielzahl an Fluidauslässen (111) zur Beaufschlagung der Mundhöhle mit dem Fluid aufweist, und
- einer Fluidzuführleitung (210), welche über ein erstes Leitungsende (211)fluidleitend mit dem Fluideinlass (121)verbunden ist und zur Zufuhr des Fluids in die Fluidkammer (130) vorgesehen ist,
**gekennzeichnet, durch**
- ein Filterelement (220), welches zur Filterung des Fluids, insbesondere des Ozon-Sauerstoff-Gemischs, mit einem zweiten Leitungsende (121) der Fluidzuführleitung (210) verbunden ist.

9. Desinfektionsset (200) nach Anspruch 8,
**dadurch gekennzeichnet, dass**
die Fluidzuführleitung (210) oder das Filterelement (220) einen Anschluss (222), insbesondere zum Anschließen einer Spritze aufweist, wobei das Fluid über den Anschluss (222) in die Fluidzuführleitung (210) des Desinfektionssets (200) einleitbar und das Filterelement (220) durchströmend der Fluidkammer (130) des Mundstücks (100) zuführbar ist.

10. Desinfektionsset (200) nach einem der Ansprüche 8 oder 9,
**dadurch gekennzeichnet, dass**
das Mundstück (100) zweiteilig ausgebildet ist, wobei ein erstes Mundstückteil (110), welches einer oberen oder einer unteren Zahnreihe der Mundhöhle zuordenbar ist und ein zweites Mundstückteil (120), welches der jeweils anderen, oberen oder unteren Zahnreihe der Mundhöhle zuordenbar ist, die Fluidkammer (130) begrenzen.

11. Desinfektionsset (200) nach Anspruch 10,
**dadurch gekennzeichnet, dass**
das erste Mundstückteil (110) zumindest teilweise als Membran ausgebildet ist und die Vielzahl an Fluidauslässen (111) aufweist, und das zweite Mundstückteil (120) als Verschalung ausgebildet ist und den Fluideinlass (121) aufweist, wobei das erste Leitungsende (211) der Fluidzuführleitung (210) in die Fluidkammer (130) mündend mit dem Fluideinlass (121) verbunden ist.
